# EUROPEAN PATENT APPLICATION

(11) **EP 4 292 579 A1**
(43) Date of publication of application: **20.12.2023**
(21) Application number: 22305868.6
(22) Date of filing: 14.06.2022
(51) Int. Cl.: A61K 8/19, A61K 8/29, A61K 8/44, A61Q 1/12, A61Q 17/04, A61K 8/02, A61K 8/73, A61Q 5/06

(54) **HYDROCOMPATIBLE LIPOPHILZED MINERAL POWDER PIGMENT COMPOSITION**

(71) Applicant: Miyoshi Europe, 69800 Saint-Priest (FR)
(72) Inventor: TAKAHASHI, Tetsuya, 69800 Saint-Priest (FR)
(74) Representative: Icosa

(57) **Abstract**

The present invention relates to a powder composition comprising composite particles, wherein the composite particles present an inorganic core, a hydrophobic coating composition and a hydrophilic coating composition, wherein the hydrophilic coating composition comprises from 20% to 60% of hydroxypropylmethylcellulose in weight relative to the total weight of the hydrophilic composition. The invention further relates to a process for obtaining such powder composition as well as cosmetic compositions comprising said powder composition.

## Description

### FIELD OF INVENTION

The present invention relates to a composition comprising surface treated mineral particles and a cosmetic composition comprising the same. The invention particularly relates to a powder having a hydrophobic and a hydrophilic surface treatment, and a cosmetic composition comprising the powder; the cosmetic composition having a dry feeling of use, a satisfactory dispersibility in aqueous media while maintaining sebum resistance and advantageous sensorial properties.

### BACKGROUND OF INVENTION

Friction, and oily skin secretions are external factors that deteriorate the durability of skin product pigment compositions. Since the surface of metal oxide pigments such as titanium or iron oxides, is hydrophilic, they tend not to adhere well to the skin and if used as such they tend to run off from the skin. Therefore, in order to improve their cosmetic durability, pigment compositions are surface treated with hydrophobic compositions in order to shield them with a hydrophobic coating.

In order to impart hydrophobicity, the surface of the particles is treated so as to be more hydrophobe prior to their use by means of constituting a hydrophobic coating. Typical hydrophobic coatings that have been reported in the art encompass silicone-based surface coating compositions and coatings with amino acids substituted with fatty acids. However, such coated particles have poor cleanability with water due to their elevated water repellency. In addition, the coated particles tend to form particle aggregates, which are not easy to disperse during the manufacturing of cosmetic formulations or even on the skin thereby leading to a negative sensorial perception during their use.

Furthermore, hydrophobically coated pigment particles cannot be formulated in stable water dispersions since their hydrophobic character leads them to phase out into a biphasic system. Thus, hydrophobically coated pigment particles can be formulated only in cosmetic emulsions.

Consequently, there is a substantial need to supply a pigment composition that addresses the aforementioned shortcomings, in particular to supply pigment compositions that do not form aggregates on the skin or in the cosmetic formulations, that can be formulated in aqueous dispersions while maintaining satisfactory sensorial properties.

The powder composition comprising composite particles according to the present invention maintains the lipophilic character of the particles towards the skin while maintaining its dispersibility in aqueous media thereby addressing the technical problems set above. Advantageously, the present composition enables ingredients that are readily available in the cosmetic market.

### SUMMARY

The present invention relates to a powder composition comprising composite particles, that present an inorganic core, a hydrophobic coating composition and a hydrophilic coating composition, wherein the hydrophilic composition comprises from 20% to 60% of hydroxypropylmethylcellulose in weight relative to the total weight of the hydrophilic composition.

According to some embodiments, the coating of the hydrophilic composition coats the coating of the hydrophobic coating composition.

In some embodiments, the powder composition according to the invention comprises in weight relative to the total weight of the powder composition:
- from 0.5% to 15% preferably from 1% to 12%, of the hydrophobic coating composition,
- from 0.5% to 10%, preferably from 3% to 8%, of the hydrophilic coating composition.

In some embodiments, the hydrophilic coating composition further comprises microcrystalline cellulose, preferably in an amount ranging from 5% to 25% in weight relative to the total weight of the hydrophilic composition.

In some embodiments, the hydrophilic coating composition further comprises from 15% to 40% in weight relative to the hydrophilic composition of an inorganic filler selected from the group consisting of magnesium carbonate, calcium carbonate, titanium dioxide, silica, silica beads, mica, talc, sericite, and mixtures thereof, preferably the inorganic filler is magnesium carbonate.

In some embodiments, the hydrophilic composition comprises in weight relative to the total weight of the hydrophilic composition:
- from 30% to 55%, preferably from 45% to 55%, of hydroxypropylmethylcellulose,
- from 5% to 25%, preferably from 8% to 12%, of microcrystalline cellulose, and
- from 15% to 40%, preferably from 25% to 35%, of magnesium carbonate.

The hydrophilic composition may further comprise at least one triglyceride, hydroxypropylcellulose or mixtures thereof.

Thus, in some embodiments, the hydrophilic composition comprises in weight relative to the total weight of the hydrophilic composition:
- from 45% to 55%, of hydroxypropylmethylcellulose,
- from 8% to 12%, of microcrystalline cellulose,
- from 25% to 35%, of magnesium carbonate,
- from 1% to 10%, of hydroxypropylcellulose, and
- from 1% to 10%, of at least one triglyceride.

According to some embodiments, the hydrophobic composition comprises:
- at least one N-acylamino amino acid or a salt thereof, wherein the acyl is a C12-C18 acyl group,
- at least a silicone surface agent, or
- mixtures thereof.

In some embodiments, the hydrophobic coating composition comprises at least one N-acylamino amino acid or a salt thereof, preferably selected from stearoyl glutamine, palmitoyl glutamine, myristoyl glutamine, sodium salts thereof, aluminum salts thereof or mixtures thereof.

Regarding the inorganic core that is coated with the aforementioned hydrophobic and hydrophilic coating compositions, it can be selected from the group consisting of titanium dioxide, red iron oxide, yellow iron oxide, black iron oxide, mica, nacre, chromium oxide, ultramarine blue, ferric blue, manganese violet, zinc oxide, aluminum oxide, zirconium oxide, boron nitride, barium sulfate or mixtures thereof.

The invention further relates to a cosmetic composition comprising the powder composition according to any one of the embodiments described hereinabove.

In some embodiments, the cosmetic composition according to the invention can be selected from a sunscreen composition, a facial make-up composition, a cosmetic foundation composition or a hair make-up composition.

Lastly, the invention relates to a process for preparing a powder composition of the invention, said process comprising the steps of:
a. Supplying a hydrophobized inorganic powder composition comprising hydrophobized composite particles presenting an inorganic core and a coating of a hydrophobic composition, then
b. Contacting the hydrophobized inorganic powder composition of step a) with a hydrophilic composition, wherein the hydrophilic composition comprises from 20% to 60% of hydroxypropylmethylcellulose in weight relative to the total weight of the hydrophilic composition; then
c. Optionally drying and/or grinding the composition obtained in step b), and
d. Recovering the powder composition according to the invention.

In some embodiments, the step a) comprises:
a1. supplying an inorganic powder composition comprising inorganic particles, then
a2. treating the inorganic particles of step a1) with a hydrophobic composition
a3. optionally drying and/or grinding the composition obtained in step a2), and
a4. recovering a hydrophobized inorganic powder composition comprising hydrophobized composite particles presenting an inorganic core and a coating of a hydrophobic composition.

### DEFINITIONS

In the present invention, the following terms have the following meanings:
**"About":** preceding a figure means plus or less 10% of the value of said figure. According to one embodiment, the term "about" preceding a figure means plus or less 5% of the value of said figure; preferably plus or less 1% of the value of said figure.
**"Essentially consisting or"** refers to a composition X comprising more than 95%, more than 96%, more than 97%, more than 98%, more than 99%, more than 99.5% of a constituent Y, in weight relative to the total weight of the composition X. "Essentially consisting of' encompasses the term "consisting of' wherein composition X is constituted 100% of constituent Y.
**"Composite"** refers to a material that is produced from at least two or more constituent materials. The constituent materials have notably dissimilar chemical or physical properties and are merged to create a material with properties unlike the individual elements. Within the finished composite structure, the individual elements remain separate and distinct, distinguishing composites from mixtures and solid solutions. In the context of the present invention, the composite particles present an inorganic core coated with an hydrophobic coating further coated with a hydroxypropylmethylcellulose-based composition.
**"Hydrophilic"** or hydrophile refers to a coating composition that is charge-polarized, typically predominantly charge-polarized, and capable of hydrogen bonding, enabling it to present an affinity with water rather than oil or other non-polar solvents. In the context of the present invention, the terms hydrophilic or hydrophile can be interchangeably used with the terms lipophobic or lipophobe.
**"Hydrophobic":** or hydrophobe refers to a coating composition that is typically unpolarized, typically predominantly unpolarized, enabling it to present an affinity with oil, fat and other non-polar solvents rather than water or other polar solvents. In the context of the present invention, the terms hydrophobic or hydrophobe can be interchangeably be used with the terms lipophilic or lipophile.
**"Pigment"** refers to a solid particle, white or colored, naturally insoluble in the liquid hydrophilic and lipophilic phases usually used in cosmetics or made insoluble by formulation in the form of lacquer, as the case may be. Typically, pigment refers to an inorganic particle or the inorganic particle core of the composite particles according to the invention.

### DETAILED DESCRIPTION

This invention relates to a powder composition comprising composite particles, wherein the composite particles present an inorganic core, a hydrophobic coating composition and a hydrophilic coating composition, wherein the hydrophilic coating composition comprises from 20% to 60% of hydroxypropylmethylcellulose in weight relative to the total weight of the hydrophilic composition.

### Inorganic core

The core of the particles of the present composition are inorganic, typically comprising, essentially consisting, or preferably consisting of metals, metal oxides, metal hydroxides or ceramic materials. In some embodiments, the inorganic core is a pigment according to any one of the inorganic pigments known in the art. It is understood that an inorganic particle, typically a pigment particle, once coated according to the present invention constitutes the inorganic core of the composite particles according to the invention.

Typically, the inorganic particles, prior to their coating according to the invention present an average diameter ranging from 5 nm to 20 µm. The average diameter can be determined by any means known in the art such as for example by sieving or laser diffraction scattering preferably by sieving.

In some embodiments, the inorganic core essentially consists of titanium dioxide (TiO₂), red iron oxide (Fe₂O₃), yellow iron oxide (FeHO₂), black iron oxide (Fe₃O₄), mica, nacre, chromium oxide (Cr₂O₃), ultramarine blue (Na₈₋₁₀Al₆Si₆O₂₄S₂₋₄ Na₈₋₁₀Al₆Si₆O₂₄S₂₋₄), ferric blue (Fe4[Fe(CN)6]3), manganese violet (H₄MnNO₇P₂), zinc oxide (ZnO), aluminum oxide (Al₂O₃), zirconium oxide (ZrO₂), boron nitride (BN) and barium sulfate (BaSO₄) or mixtures thereof.

In some embodiments, the inorganic core essentially consists of metal oxides or hydroxides, preferably selected from titanium dioxide (TiO₂); iron oxides and hydroxides such as red iron oxide (Fe₂O₃), yellow iron oxide (FeHO₂), black iron oxide (Fe₃O₄) or mixtures thereof ; chromium oxide (Cr₂O₃), ultramarine blue (Na₈₋₁₀Al₆Si₆O₂₄S₂₋₄ Na₈₋₁₀Al₆Si₆O₂₄S₂₋₄), ferric blue (Fe4[Fe(CN)6]3), manganese violet (H₄MnNO₇P₂), zinc oxide (ZnO), aluminum oxide (Al₂O₃), zirconium oxide (ZrO₂), or mixtures thereof.

Preferably, the inorganic core essentially consists of titanium dioxide (TiO₂), iron oxides and hydroxides, or mixtures thereof, typically the iron oxides and hydroxides being selected from red iron oxide (Fe₂O₃), yellow iron oxide (FeHO₂), black iron oxide (Fe₃O₄),and mixtures thereof. In some embodiments, the inorganic core essentially consists of red iron oxide (Fe₂O₃). In some embodiments, the inorganic core essentially consists of yellow iron oxide (FeHO₂). In some embodiments, the inorganic core essentially consists of black iron oxide (Fe₃O₄). In some embodiments, the inorganic core essentially consists of a mixture of iron oxides and/or iron hydroxides selected from red iron oxide (Fe₂O₃), yellow iron oxide (FeHO₂), black iron oxide (Fe₃O₄). In some embodiments, the inorganic core of the particles essentially consists of iron oxides and hydroxides or mixtures thereof and the particles present an average diameter ranging from 5 µm to 20 µm, from 8 µm to 15µm, or about 10 µm, prior to their coating.

In some preferred embodiments, the inorganic core essentially consists of titanium dioxide (TiO₂). In some embodiments, the inorganic core of the particles essentially consists of titanium dioxide (TiO₂) and the particles present an average diameter ranging from 100 nm to 1 µm, from 200 nm to 600 nm, or about 500 nm, prior to their coating.

In some embodiments, the inorganic core essentially consists of mica, i.e. a phyllosilicate compound of formula X₂Y₄₋₆Z₈O₂₀(OH, F)4, wherein X is K, Na, Ca Ba, Rb, or Cs, preferably K, Na or Ca; Y is Al, Mg, Fe Mn, Cr, Ti, or Li, preferably Al, Mg, or Fe; Z is Si, Al, Fe³⁺ or Ti, preferably Z is Si or Al.

In some embodiments, the inorganic core essentially consists of nacre, a calcium carbonate based, typically aragonite based inorganic compound further comprising an organic matrix of elastic biopolymers such as chitin, lustrin and silk-like proteins.

According to the invention, the inorganic particles are coated with a hydrophobic coating composition and a hydrophilic coating composition comprising from 20% to 60% of hydroxypropylmethylcellulose in weight relative to the hydrophilic composition.

According to a first variant, the hydrophilic coating composition coats the hydrophobic coating composition. According to such variant, the hydrophobic coating composition coats the inorganic particle core, said hydrophobic coating composition being between the particle core and the hydrophilic coating composition comprising from 20% to 60% of hydroxypropylmethylcellulose in weight relative to the total weight of the hydrophilic composition.

According to a second variant, the hydrophobic coating composition coats the hydrophilic coating composition comprising from 20% to 60% of hydroxypropylmethylcellulose in weight relative to the total weight of the hydrophilic composition. According to such variant, the hydrophilic coating composition coats the inorganic particle core, said hydrophilic coating composition being between the particle core and the hydrophobic coating composition.

Preferably, the inorganic particle cores are present, in the powder composition, in an amount ranging from 30% to 98% by weight, in particular from 40% to 95% by weight, preferably from 70% to 93%, and more preferentially from 70% to 89% by weight, relative to the total weight of the powder composition.

### Hydrophobic coating composition

The surface of metal oxide pigments such as titanium / iron oxides or iron hydroxides, that can be used according to the present invention, is hydrophilic. Thus, they tend not to adhere well to the skin and if used as such they tend to run off from the skin. According to some embodiments, any surface treatment composition known in the art to confer a hydrophobic coating to a particle's surface can be applied in the context of the present invention.

Advantageously, the hydrophobic coating composition comprises or essentially consists of at least one compound chosen from silicone surface agents; N- acylamino acids or salts thereof; fluoro surface agents; metal soaps; lecithin and derivatives thereof; isopropyl triisostearyl titanate; isostearyl sebacate; natural plant or animal waxes; polar synthetic waxes; fatty esters; phospholipids; and mixtures thereof, preferably of at least one compound chosen from silicone surface agents; N- acylamino acids or salts thereof, and mixtures thereof, more preferably the hydrophobic coating composition comprises or essentially consists of at least one compound chosen from silicone surface agents, in particular at least one polydimethylsiloxane such as triethoxysilylethyl polydimethylsiloxyethyl dimethicone; N- acylamino acids or salts thereof further selected from stearoyl glutamine, palmitoyl glutamine sodium salts thereof, aluminum salts thereof, and mixtures thereof.

In some embodiments, the hydrophobic coating composition comprises or essentially consists of at least one compound chosen from fluoro surface agents; metal soaps, such as zinc laurate, magnesium stearate, magnesium myristate and zinc stearate, and mixtures thereof; lecithin and derivatives thereof; isopropyl triisostearyl titanate; isostearyl sebacate; natural plant or animal waxes such as beeswax, lanolin wax, orange wax, lemon wax and Chinese insect waxes, rice bran wax, carnauba wax, candelilla wax, ouricury wax, cork fiber wax, sugarcane wax, Japan wax, sumac wax and montan wax; polar synthetic waxes; fatty esters such as jojoba esters; phospholipids; and mixtures thereof.

In some embodiments, the hydrophobic coating composition is in an amount ranging from 0.5% to 15% in weight relative to the total weight of the powder composition. In some embodiments, the hydrophobic coating composition is in an amount ranging from 0.5% to 5% in weight relative to the total weight of the powder composition. In some embodiments, the hydrophobic coating composition is in an amount ranging from 1% to 12% in weight relative to the total weight of the powder composition. In some embodiments, the hydrophobic coating composition is in an amount ranging from 1% to 5%, such as for example about 3%, in weight relative to the total weight of the powder composition.

Advantageously, the mass ratio of the amount of the first coating of a hydrophobic composition/ and the amount of the hydrophilic coating composition is equal or preferably inferior to 1, more preferably ranging from 0,1 to 1, even more preferably ranging from 0.3 to 0.8, such as for example about 0.4 or about 0.7.

According to one variant, the hydrophobic coating composition is covalently or non-covalently, more preferably non-covalently bonded to the surface of the hydrophilic coating composition. According to a preferred variant, the hydrophobic coating composition is covalently or non-covalently, preferably covalently bonded to the surface of the inorganic core of the particles.

### Silicone surface agent

In some embodiments, the hydrophobic coating composition comprises or essentially consists of at least one silicone surface agent. The silicone surface agents may be chosen from silane derivatives such as organopolysiloxanes, silicone-acrylate copolymers, silicone resins, and mixtures thereof.

In some embodiments, the hydrophobic coating composition comprises or essentially consists of
- at least one organopolysiloxane, such as at least one polydimethylsiloxane, at least one polymethylhydrogenosiloxane and/or at least one poly alkoxy dimethylsiloxane;
- at least one non-elastomeric organopolysiloxane, especially chosen from at least one polydimethylsiloxane such as triethoxysilylethyl polydimethylsiloxyethyl dimethicone;
- at least one alkylsilane;
- at least one alkoxysilane such as at least one alkyltriethoxy silane and/or at least one alkyltrimethoxysilane;
- at least one silicone-acrylate polymer;
- at least one silicone resin;
- at least one fluorosilicone; or
- a mixture thereof,
wherein the alkyl group (R) represents methyl, ethyl, propyl, butyl or octyl, 2-phenylethyl, 2-phenylpropyl or 3,3,3-trifluoropropyl radicals, aryl radicals such as phenyl, tolyl or xylyl, or substituted aryl radicals such as phenylethyl, wherein the alkoxy group (R-O) represents methoxy, ethoxy, propoxy, butyl-oxy or octyl-oxy, 2-phenylethyl-oxy, 2-phenylpropyl-oxy or 3,3,3-trifluoropropyl-oxy radicals, aryl-oxy radicals such as phenoxy, tolyl-oxy or xylyl-oxy, or substituted aryl-oxy radicals such as phenylethyl-oxy.

Methods for surface-treating pigments with a silicone-based compounds, in particular silicone surface agents, are generally known in the art. For instance, the inorganic particles can be dispersed in an organic solvent where the silicone-based compound is added. Upon heating the mixture, covalent bonds are created between the silicone compound and the surface of the pigment, thereby leading to an inorganic composition wherein the inorganic particles form a core that is coated with a coating of the silicone-based compound.

In some embodiments, the hydrophobic coating composition comprises or essentially consists of at least one organopolysiloxane. The term "organopolysiloxane compound" means a compound having a structure comprising an alternance of silicon atoms and oxygen atoms and comprising organic radicals linked to silicon atoms.

In some embodiments, the hydrophobic coating composition comprises or essentially consists of at least one organopolysiloxane selected from polydimethylsiloxanes, polymethylhydrogenosiloxanes and poly alkoxy dimethylsiloxanes.

In some embodiments, the hydrophobic coating composition comprises or essentially consists of at least one poly alkoxy dimethylsiloxanes, wherein the alkoxy group may selected from methoxy, ethoxy, propoxy, butyl-oxy or octyl-oxy, 2-phenylethyl-oxy, 2-phenylpropyl-oxy or 3,3,3-trifluoropropyl-oxy radicals, aryl-oxy radicals such as phenoxy, tolyl-oxy or xylyl-oxy, or substituted aryl-oxy radicals such as phenylethyl-oxy.

According to some embodiments, the hydrophobic coating composition comprises or essentially consists of at least one non-elastomeric organopolysiloxane, especially selected from polydimethylsiloxanes. According to a specific embodiment, the hydrophobic coating composition comprises or essentially consists of triethoxysilylethyl polydimethylsiloxyethyl dimethicone.

According to some embodiments, the hydrophobic coating composition comprises or essentially consists of at least one alkyltriethoxysilane or at least one alkyltrimethoxysilane.

According to some embodiments, the hydrophobic coating composition comprises or essentially consists of at least one silicone-acrylate polymer.

In some embodiments, the at least one silicone-acrylate polymer is a silicone polymer of formula (I): 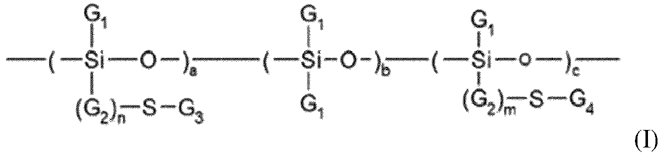 in which the radicals G₁, which may be identical or different, represent hydrogen or a C₁-C₁₀ alkyl radical or alternatively a phenyl radical; the radicals G₂, which may be identical or different, represent a C₁-C₁₀ alkylene group; G₃ represents a polymeric residue resulting from the (homo)polymerization of at least one ethylenically unsaturated anionic monomer; G₄ represents a polymeric residue resulting from the (homo)polymerization of at least one ethylenically unsaturated hydrophobic monomer; m and n are equal to 0 or 1; a is an integer ranging from 0 to 50; b is an integer that may be between 10 and 350, c is an integer ranging from 0 to 50; with the proviso that one of the parameters a and c is other than 0.

In some embodiments:
- the radicals G₁ denote an alkyl radical, preferably a methyl radical;
- n is non-zero, and the radicals G₂ represent a divalent C₁-C₃ radical, preferably a propylene radical;
- G₃ represents a polymeric radical resulting from the (homo)polymerization of at least one monomer of the ethylenically unsaturated carboxylic acid type, preferably acrylic acid and/or methacrylic acid;
- G₄ represents a polymeric radical resulting from the (homo)polymerization of at least one monomer of the (C₁-C₁₀)alkyl (meth)acrylate type, preferably such as isobutyl or methyl (meth)acrylate.

In some embodiments, silicone polymers corresponding to formula (I) are especially polydimethylsiloxanes (PDMS) onto which are grafted, via a connecting chain unit of thiopropylene type, polymer units of the polyisobutyl (meth)acrylate type.

Examples of silicone polymers corresponding to formula (I) are especially polydimethylsiloxanes (PDMS) onto which are grafted, via a connecting chain unit of thiopropylene type, mixed polymer units of the poly(meth)acrylic acid type and of the polymethyl (meth)acrylate type or of the polyisobutyl (meth)acrylate type.

According to some embodiments, the hydrophobic coating compositionaccording to the invention comprises or essentially consists of at least one silicone-resin.

The term "resin" means a three-dimensional structure. The silicone resins may be soluble or swellable in silicone oils. These resins are crosslinked polyorganosiloxane polymers.

The nomenclature of silicone resins is known under the name "MDTQ", the resin being described as a function of the various siloxane monomer units that it comprises, each of the letters "MDTQ" characterizing a type of unit.

The letter M represents the mono functional unit of formula (CH₃)₃SiO_{1/2}, the silicon atom being bonded to only one oxygen atom in the polymer comprising this unit. The letter D means a difunctional unit (CH₃)₂SiO_{2/2} in which the silicon atom is bonded to two oxygen atoms. The letter T represents a trifunctional unit of formula (CH₃)SiO_{3/2}. In the units M, D and T defined above, at least one of the methyl groups may be substituted with a group R other than a methyl group, such as a hydrocarbon-based radical (especially alkyl) containing from 2 to 10 carbon atoms or a phenyl group, or alternatively a hydroxy 1 group. Finally, the letter Q means a tetrafunctional unit SiO_{4/2} in which the silicon atom is bonded to four hydrogen atoms, which are themselves bonded to the rest of the polymer.

Various resins with different properties may be obtained from these different units, the properties of these polymers varying as a function of the type of monomers (or units), of the type and number of substituted radicals, of the length of the polymer chain, of the degree of branching and of the size of the side chains.

According to some embodiments, the at least one silicone-resin is selected from:
- siloxysilicates, which may be trimethyl siloxysilicates of formula [(CH₃)₃XSiXO]ₓX(SiO_{4/2})_{y} (MQ units) in which x and y are integers ranging from 50 to 80;
- polysilsesquioxanes of formula (CH₃SiO_{3/2})ₓ (T units) in which x is greater than 100 and at least one of the methyl radicals of which may be substituted with a group R as defined above;
- polymethylsilsesquioxanes, which are polysilsesquioxanes in which none of the methyl radicals is substituted with another group.

According to some embodiments, the hydrophobic coating composition comprises or essentially consists of at least one fluorosilicone.

In some embodiments, the at least one fluorosilicone compound may be chosen from perfluoroalkyl dimethicones, perfluoroalkyl silanes and perfluoroalkyl trialkoxysilanes.

In some embodiments, the at least one fluorosilicone compound is a perfluoroalkyl dimethicones of formula II: 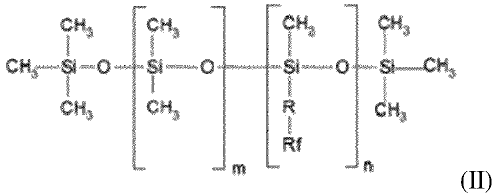 in which:
- R represents a linear or branched divalent alkyl group containing from 1 to 6 carbon atoms, preferably a divalent methyl, ethyl, propyl or butyl group;
- Rf represents a perfluoroalkyl radical containing 1 to 9 carbon atoms and preferably 1 to 4 carbon atoms;
- m is chosen between 0 and 150 and preferably from 20 to 100; and
- n is chosen between 1 and 300 and preferably from 1 to 100.

### N-acylamino acids or salts thereof

In some preferred embodiments, the hydrophobic coating composition comprises or essentially consists of at least one N-acylamino acid or a salt thereof.

N-acylamino acids or salts thereof as described herein may comprise an acyl group containing from 8 to 22 carbon atoms, for instance a 2-ethylhexanoyl, caproyl, lauroyl, myristoyl, palmitoyl, stearoyl or cocoyl group, preferably the acyl group contains from 12 to 22 carbon atoms. In some embodiments, the acyl group is selected from myristoyl, palmitoyl, and stearoyl. In some embodiments, the acyl group is selected from myristoyl, and palmitoyl. In some embodiments, the acyl group is myristoyl. In some embodiments, the acyl group is palmitoyl. In some embodiments, the acyl group stearoyl.

In some embodiments, the amino acid is selected from lysine, glutamic acid or alanine. In some embodiments, the amino acid is glutamic acid.

In some embodiments, the salts of N-acylamino acids may be the aluminum, magnesium, calcium, zirconium, zinc, sodium or potassium salts. In some embodiments, the salts of N-acylamino acids may be the aluminum, and/ or sodium salts.

In some embodiments, the hydrophobic coating composition comprises or essentially consists of at least one N-acylamino amino acid or a salt thereof, preferably selected from stearoyl glutamine, palmitoyl glutamine sodium salts thereof, aluminum salts thereof or mixtures thereof.

In some embodiments, the hydrophobic coating composition comprises or essentially consists of stearoyl glutamate, typically aluminum and/or sodium stearoyl glutamate.

In some embodiments, the hydrophobic coating composition comprises or essentially consists of myristoyl glutamate, typically aluminum and/or sodium myristoyl glutamate. In some embodiments, the hydrophobic coating compositionaccording to the invention comprises or essentially consists of myristoyl glutamate, typically aluminum and/or sodium myristoyl glutamate is a coating of the VAA^{®}, VAI^{®} or MiyoNAT VAA@ product series commercialized by MIOSHI.

In some embodiments, the hydrophobic coating composition comprises or essentially consists of palmitoyl glutamate, typically aluminum and/or sodium palmitoyl glutamate such as for example aluminum palmitoyl glutamate commercialized under the reference NAI^{®} product series commercialized by MIOSHI.

### Fluoro surface agent

In some embodiments, the hydrophobic coating composition comprises or essentially consists of at least one fluoro surface agent. The fluoro surface agent may designate fluorinated organic compounds.

The fluoro surface agents may be chosen from perfluoroalkyl phosphates, perfluoropolyethers, polytetrafluoropolyethylenes (PTFE), perfluoroalkanes, perfluoroalkyl silazanes, polyhexafluoropropylene oxides, and polyorganosiloxanes comprising perfluoroalkyl perfluoropolyether groups. The term "perfluoroalkyl radical" means an alkyl radical in which all the hydrogen atoms have been replaced with fluorine atoms.

Among the linear perfluoroalkanes that may be mentioned are perfluorocycloalkanes, perfluoro(alkylcycloalkanes), perfluoropolycycloalkanes, aromatic perfluoro hydrocarbons (perfluoroarenes) and hydrocarbon-based perfluoro organic compounds comprising at least one heteroatom.

Among the perfluoroalkanes, mention may be made of the linear alkane series such as perfluorooctane, perfluorononane or perfluorodecane.

Among the perfluorocycloalkanes and perfluoro(alkylcycloalkanes), mention may be made of perfluorodecalin, perfluoro(methyldecalin) and perfluoro(C₃-C₅ alkylcyclohexanes), such as perfluoro(butylcyclohexane).

Among the perfluoropolycycloalkanes, mention may be made of bicyclo[3.3.1]nonane derivatives such as perfluorotrimethylbicyclo[3.3.1]nonane, adamantane derivatives such as perfluorodimethyladamantane, and hydrogenated perfluorophenanthrene derivatives, such as tetracosafluorotetradecahydrophenanthrene.

Among the perfluoroarenes, mention may be made of perfluoronaphthalene derivatives, for instance perfluoronaphthalene and perfluoromethyl-1 -naphthalene.

### Hydrophilic coating composition

Inorganic particles that are only surface treated so as to present a hydrophobic coating surrounding the inorganic core, tend to agglomerate with each other, are not easy to disperse in aqueous media during the manufacturing of cosmetic formulations and can lead to a negative sensorial perception, such as a feeling of "heaviness", during their use. Furthermore, they are not compatible for aqueous cosmetic formulations such as aqueous dispersions.

Thus, the composite particles of the powder composition of the invention present an inorganic core and a hydrophobic coating composition as described above, and they further comprise a hydrophilic coating composition comprising from 20% to 60% of hydroxypropylmethylcellulose (HPMC) in weight relative to the total weight of the hydrophilic composition. In some embodiments, the hydrophilic coating composition comprises from 30% to 55% of HPMC in weight relative to the total weight of the hydrophilic composition. In some embodiments, the hydrophilic coating composition comprises from 40% to 50% of HPMC in weight relative to total weight of the hydrophilic composition. In some embodiments, the hydrophilic coating composition comprises about 45%, about 50%, or about 55%, preferably about 50% of HPMC in weight relative to total weight of the hydrophilic composition.

In some embodiments, hydrophilic coating composition is in an amount ranging between 0.5% and 10% in weight relative to the total weight of the powder composition. In some embodiments, the hydrophilic coating composition is in an amount ranging between 3% and 8% in weight relative to the total weight of the powder composition.

In some embodiments, the hydrophilic coating composition is in an amount ranging between 7% and 9%, such as for example about 8%, in weight relative to the total weight of the powder composition.

In some embodiments, the hydrophilic coating composition is in an amount ranging between 3% and 5%, such as for example about 4%, in weight relative to the total weight of the powder composition.

In some embodiments, the hydrophilic coating composition is in an amount ranging between 2% and 4%, typically about 3% in weight relative to the total weight of the inorganic particles coated with the hydrophobic coating composition.

According to one variant, the hydrophilic coating composition is covalently or non-covalently, preferably non-covalently bonded to the surface of the inorganic core of the particles. According to a preferred variant, the hydrophilic coating composition is covalently or non-covalently, more preferably non-covalently bonded to the surface of the hydrophobic coating composition.

### Hydroxypropylmethylcellulose

Hydroxypropylmethylcellulose (HPMC), also known as hypromellose is nonionic cellulose ether, typically made from natural cotton fiber under series of chemical processing by etherification of the cellulose moieties of the cellulose backbone structure having the CAS n° 9004-65-3.

It is an odorless, tasteless and non-toxic white powder that can be dissolved in cold water to form a transparent viscous solution with the gelling and/or thickening properties. HPMC can typically present a methoxy substitution of the hydroxyl moieties of the cellulose backbone in an amount ranging from 15.0% to 30.0%, typically from 19.0% to 30.0%, preferably from 27.0% to 30.0% or from 19.0% to 27.0%, in weight relative to total weight of the HPMC composition. In some embodiments, the HPMC presents a methoxy substitution ranging from 27.0% to 30.0% in weight relative to total weight of the HPMC composition.

HPMC can typically present a hydroxypropyl substitution of the hydroxyl moieties of the cellulose backbone in an amount ranging from 2.0% to 15.0%, typically from 4.0% to 12.0%, preferably from 4.0% to 7.5% or from 7.0% to 12.0%, in weight relative to total weight of the HPMC composition. In some embodiments, HPMC presents a hydroxypropyl substitution ranging from 7.0% to 12.0% in weight relative to total weight of the HPMC composition, such as for example HPMC VIVAPHARM^{®} HPMC E6 commercialized by JRS Pharma.

### Microcrystalline cellulose

According to some embodiments, the hydrophilic composition may further comprise microcrystalline cellulose (MCC), preferably in an amount ranging from 5% to 25% in weight relative to the hydrophilic composition. In some embodiments, the hydrophilic coating composition comprises about 5%, about 10%, or about 15%, preferably about 10% of microcrystalline cellulose in weight relative to total weight of the hydrophilic composition.

Microcrystalline cellulose (MCC) is a naturally occurring polymer composed of glucose units connected by a 1-4 beta glycosidic bond and that is typically used in cosmetics as an abrasive, absorbent, anti-caking agent, aqueous viscosity increasing agent, binder, bulking agent, emulsion stabilizer, slip modifier, and texturizer. Typically, the degree of polymerization is typically less than 400 and the MCC particles with average size lower than 5 µm are less than 10% in weight relative to total weight of the MCC composition, as defined by sieving method such as for example Air Jet sieve.

According to some embodiments, the MCC presents at most 10%, preferably at most 8% particles of average size exceeding 32 µm, as defined by sieving method such as for example Air Jet sieve, such as for example MCC commercialized by JRS Pharma as VIVAPUR^{®} 105.

### Inorganic filler

According to some embodiments, the hydrophilic composition further comprises an inorganic filler, preferably in an amount ranging from 25% to 35% in weight relative to total weight of the hydrophilic composition. Typically, the inorganic filler acts as a bulking agent of the hydrophilic coating composition.

In some embodiments, the inorganic filler is selected from the group consisting of magnesium carbonate, calcium carbonate, titanium dioxide, silica, silica beads, mica, talc, sericite and a mixture thereof, preferably the inorganic filler is magnesium carbonate.

### Hydroxypropylcellulose

According to some embodiments, the hydrophilic composition further comprises hydroxypropylcellulose, preferably in an amount ranging from 1% to 20%, from 5% to 15%, from 1% to 10%, preferably from 5% to 10%, typically about 5%, in weight relative to total weight of the hydrophilic composition. Hydroxypropylcellulose (HPC) is nonionic cellulose ether, that is typically used in pharmaceutical compositions as a binder. Typically, the molar substitution degree of the glucose monomers by hydroxypropyl moieties ranges from 2 to 4.0. In some embodiments, the HPC presents a hydroxypropyl substitution ranging from more than 14%, more than 15% or more than 20% in weight relative to total weight of the HPC composition.

### Triglyceride

According to some embodiments, the hydrophilic composition may further comprise at least one triglyceride, preferably in an amount ranging from 1% to 20%, from 5% to 15%, from 1% to 10%, preferably from 5% to 10% in weight relative to total weight of the hydrophilic composition. Triglycerides are esters derived from glycerol esterified with three fatty acids, typically C4-C12 carbon fatty acids. In some embodiments, the at least one triglyceride is at least one medium-chain triglyceride, wherein the fatty acids are a C6-C12 carbon fatty acids. In some embodiments, the at least one triglyceride is a commercially available Miglyol^{®} mixture of triglycerides. In some embodiments, the at least one triglyceride is at least one medium-chain triglyceride, wherein the fatty acids of the medium-chain triglyceride are selected from caprylic (C8), capric (C10) caproic (C6) and lauric acid (C12) and a mixture thereof.

In some embodiments, the at least one triglyceride is a mixture of medium-chain triglycerides comprising or essentially consisting of caprylic acid (C8) in an amount ranging from 50% to 65%, capric acid (C10) in an amount ranging from 30% to 45%, caproic acid (C6) in an amount ranging from more than 0% to 2% and lauric acid (C12) in an amount ranging from more than 0% to 3%, the percentages being expressed in weight relative to total weight of the medium-chain triglyceride mixture, such as for example miglyol 812@ commercialized by Condea Chemie GmbH.

According to some embodiments, the hydrophilic coating composition comprises, in weight relative to the total weight of the hydrophilic coating composition:
- from 20% to 60%, from 30% to 55%, preferably from 45% to 55%, of hydroxypropylmethylcellulose (HPMC);
- from 5% to 25%, preferably from 8% to 12%, of microcrystalline cellulose (MCC), and
- from 15% to 40%, preferably from 25% to 35%, an inorganic filler composition preferably magnesium carbonate.

According to some embodiments, the hydrophilic coating composition comprises, in weight relative to the total weight of the hydrophilic coating composition:
- from 30% to 55%, preferably from 45% to 55%, of hydroxypropylmethylcellulose (HPMC), preferably the HPMC presenting a methoxy substitution ranging from 27.0% to 30.0% in weight relative to the HPMC composition and/or a hydroxypropyl substitution ranging from 7.0% to 12.0% in weight relative to the HPMC composition;
- from 5% to 25%, preferably from 8% to 12%, of microcrystalline cellulose,
- from 15% to 40%, preferably from 25% to 35%, an inorganic filler composition preferably magnesium carbonate;
- from 5% to 15%, from 1% to 10%, preferably from 5% to 10% of hydroxypropylcellulose (HPC); and
- from 1% to 20%, from 1% to 10%, preferably from 5% to 15%, more preferably from 5% to 10% of at least one triglyceride, preferably at least one medium-chain triglyceride, such as Miglyol 812@.

According to some embodiments, the hydrophilic coating composition comprises, in weight relative to the total weight of the hydrophilic coating composition:
- from 45% to 55%, of hydroxypropylmethylcellulose, preferably the HPMC presenting a methoxy substitution ranging from 27.0% to 30.0% in weight relative to the HPMC composition and/or a hydroxypropyl substitution ranging from 7.0% to 12.0% in weight relative to the HPMC composition
- from 8% to 12%, of microcrystalline cellulose,
- from 25% to 35%, of magnesium carbonate,
- from 1% to 10%, of hydroxypropylcellulose, and
- from 1% to 10%, of at least one triglyceride, preferably at least one medium-chain triglyceride, such as Miglyol 812^{®}.

According to some embodiments, the powder composition comprises by weight, relative to the total weight of the composition:
a) from 30% to 98%, preferably from 70% to 93% inorganic particle cores as described above;
b) from 0.5% to 15% preferably from 1% to 12%, from 1% to 5%, or from 2% to 4% of a hydrophobic coating composition, as described above, and
c) from 0.5% to 10%, preferably from 3% to 8% of a hydrophilic coating composition, as described above, with the proviso that the sum of the amounts of a)-c) do not exceed 100%.

According to some embodiments, the powder composition comprises by weight, relative to the total weight of the composition:
a) from 30% to 98%, preferably from 70% to 93% inorganic particle cores as described above;
b) from 0.5% to 15% preferably from 1% to 12%, from 1% to 5%, or from 2% to 4% of a hydrophobic coating composition, as described above, preferably selected from at least one compound of silicone surface agents, N- acylamino acids or salts thereof, and mixtures thereof; and
c) from 0.5% to 15%, preferably from 3% to 8% of a hydrophilic coating composition, as described above, preferably the hydrophilic composition comprises hydrophilic coating composition comprises, in weight relative to the total weight of the hydrophilic coating composition:
   - from 20% to 60%, from 30% to 55%, preferably from 45% to 55%, of hydroxypropylmethylcellulose (HPMC);
   - from 5% to 25%, preferably from 8% to 12%, of microcrystalline cellulose (MCC), and
   - from 15% to 40%, preferably from 25% to 35%, an inorganic filler composition preferably magnesium carbonate,
   with the proviso that the sum of the amounts of a)-c) do not exceed 100%.

According to some embodiments, the powder composition comprises by weight, relative to the total weight of the composition:
a) from 30% to 98%, preferably from 70% to 93% inorganic particle cores as described above;
b) from 0.5% to 15% preferably from 1% to 12%, from 1% to 5%, or from 2% to 4% of a hydrophobic coating composition, as described above, preferably selected from at least one compound chosen from silicone surface agent, in particular at least one polydimethylsiloxane such as triethoxysilylethyl polydimethylsiloxyethyl dimethicone; at least one N- acylamino acid, wherein the acyl is a C12-C18 acyl group or salts thereof, preferably further selected from stearoyl glutamine, palmitoyl glutamine, myristoyl glutamine, sodium salts thereof, aluminum salts thereof, and mixtures thereof; and
c) from 0.5% to 15%, preferably from 3% to 8% of a hydrophilic coating composition, as described above, preferably the hydrophilic composition comprises hydrophilic coating composition comprises, in weight relative to the total weight of the hydrophilic coating composition:
   - from 20% to 60%, from 30% to 55%, preferably from 45% to 55%, of hydroxypropylmethylcellulose (HPMC);
   - from 5% to 25%, preferably from 8% to 12%, of microcrystalline cellulose (MCC), and
   - from 15% to 40%, preferably from 25% to 35%, an inorganic filler composition preferably magnesium carbonate,
   with the proviso that the sum of the amounts of a)-c) do not exceed 100%.

According to some embodiments, the powder composition comprises by weight, relative to the total weight of the composition:
a) from 30% to 98%, preferably from 70% to 93% inorganic particle cores as described above;
b) from 0.5% to 15% preferably from 1% to 12%, from 1% to 5%, or from 2% to 4% of a hydrophobic coating composition essentially consisting of N-acylamino acids or salts thereof, and mixtures thereof, preferably selected from aluminum and/or sodium myristoyl glutamate, stearoyl glutamate, palmitoyl glutamate, and mixtures thereof,
c) from 0.5% to 10%, preferably from 3% to 8% of a hydrophilic coating composition, as described above, preferably the hydrophilic coating composition comprises, in weight relative to the total weight the hydrophilic coating composition:
   - from 20% to 60%, from 30% to 55%, preferably from 45% to 55%, of hydroxypropylmethylcellulose (HPMC);
   - from 5% to 25%, preferably from 8% to 12%, of microcrystalline cellulose (MCC), and
   - from 15% to 40%, preferably from 25% to 35%, an inorganic filler composition preferably magnesium carbonate,
   with the proviso that the sum of the amounts of a)-c) do not exceed 100%.

According to some embodiments, the powder composition comprises by weight, relative to the total weight of the composition:
a) from 30% to 98%, preferably from 70% to 93% inorganic particle cores as described above, preferably selected from
   - inorganic particles essentially consisting of iron oxides and hydroxides selected from red iron oxide (Fe₂O₃), yellow iron oxide (FeHO₂), black iron oxide (Fe₃O₄), and mixtures thereof, and/or
   - inorganic particles essentially consisting of titanium dioxide (TiO₂);
b) from 0.5% to 15% preferably from 1% to 12%, from 1% to 5%, or from 2% to 4% of a hydrophobic coating composition essentially consisting of at least one polydimethylsiloxane such as triethoxysilylethyl polydimethylsiloxyethyl dimethicone,
c) from 0.5% to 10%, preferably from 3% to 8% of a hydrophilic coating composition, as described above, preferably the hydrophilic coating composition comprises, in weight relative to the total weight of the hydrophilic coating composition:
   - from 30% to 55%, preferably from 45% to 55%, of hydroxypropylmethylcellulose (HPMC), preferably the HPMC presenting a methoxy substitution ranging from 27.0% to 30.0% in weight relative to the HPMC composition and/or a hydroxypropyl substitution ranging from 7.0% to 12.0% in weight relative to the HPMC composition;
   - from 5% to 25%, preferably from 8% to 12%, of microcrystalline cellulose,
   - from 15% to 40%, preferably from 25% to 35%, an inorganic filler composition preferably magnesium carbonate;
   - from 5% to 15%, preferably from 5% to 10% of hydroxypropylcellulose (HPC); and
   - from 1% to 20%, from 5% to 15%, preferably from 5% to 10% of at least one triglyceride, preferably at least one medium-chain triglyceride, such as Miglyol 812@,
   with the proviso that the sum of the amounts of a)-c) do not exceed 100%.

According to some embodiments, the powder composition comprises by weight, relative to the total weight of the composition:
a) from 30% to 98%, preferably from 70% to 93% inorganic particle cores as described above, preferably selected from
   - inorganic particles essentially consisting of iron oxides and hydroxides selected from red iron oxide (Fe₂O₃), yellow iron oxide (FeHO₂), black iron oxide (Fe₃O₄), and mixtures thereof, and/or
   - inorganic particles essentially consisting of titanium dioxide (TiO₂);
b) from 0.5% to 15% preferably from 1% to 12%, from 1% to 5%, or from 2% to 4% of a hydrophobic coating composition essentially consisting of N- acylamino acids or salts thereof, and mixtures thereof, preferably selected from stearoyl glutamine, palmitoyl glutamine, myristoyl glutamine, sodium salts thereof, aluminum salts thereof or mixtures thereof even more preferably selected from aluminum and/or sodium myristoyl glutamate,
c) from 0.5% to 10%, preferably from 3% to 8% of a hydrophilic coating composition, as described above, preferably the hydrophilic coating composition comprises, in weight relative to the total weight of the hydrophilic coating composition:
   - from 30% to 55%, preferably from 45% to 55%, of hydroxypropylmethylcellulose (HPMC), preferably the HPMC presenting a methoxy substitution ranging from 27.0% to 30.0% in weight relative to the HPMC composition and/or a hydroxypropyl substitution ranging from 7.0% to 12.0% in weight relative to the HPMC composition;
   - from 5% to 25%, preferably from 8% to 12%, of microcrystalline cellulose,
   - from 15% to 40%, preferably from 25% to 35%, an inorganic filler composition preferably magnesium carbonate;
   - from 5% to 15%, preferably from 5% to 10% of hydroxypropylcellulose (HPC); and
   - from 1% to 20%, from 5% to 15%, preferably from 5% to 10% of at least one triglyceride, preferably at least one medium-chain triglyceride, such as Miglyol 812@,
   with the proviso that the sum of the amounts of a)-c) do not exceed 100%.

The invention further relates to a cosmetic composition comprising the powder composition according to any one the hereinabove described embodiments.

In some embodiments, the cosmetic composition comprises from 1 % to 99%, from 5 % to 85%, from 10 % to 75%, from or 20 to 60%, of the powder composition according to the invention, in weight relative to the total weight of the cosmetic composition. In some embodiments, the cosmetic composition essentially consists of the powder composition according to the invention.

In some embodiments, the cosmetic composition comprises the powder composition according to the invention in association with at least one cosmetically acceptable ingredient. Cosmetically, acceptable ingredients refer to the excipients or cosmetically active compounds that are appropriate for such uses and do not provoke any side effects such as toxicity, irritation, inflammation or allergic response. Such excipients may be selected from bulking agents, fillers, diluents that may facilitate the production, application or absorption of a cosmetical composition, dyes, coatings, dispersants, anti-tacking agents, preservatives or flavors. In the context of the present invention a cosmetically acceptable excipients also refer to personal-care product acceptable ingredients. The present powder composition may be of particular use in the formulation of cosmetically or pharmaceutically active compounds previously disclosed in the art. Pharmaceutically or therapeutically active compound refers to an active principle that is suitable for therapeutic use, and relates to health. A cosmetically active compound refers to an active principle, optionally in association with a cosmetically acceptable vehicle or excipient, leading to a cosmetic composition. The use of a cosmetic composition does not encompass therapeutic uses, and relates to well-being and beauty.

In some embodiments, the cosmetic composition according to the invention can be selected from a sunscreen composition, a facial make-up composition, a cosmetic foundation composition or a hair make-up composition. According to some embodiments, the cosmetic composition is in the form of a dispersion of the powder of the invention in cosmetically acceptable aqueous media such as water or water in association with at least one hydrosoluble and cosmetically acceptable ingredient.

The invention further relates to the use of the powder composition according to the invention for the manufacture of a cosmetic composition. The invention also relates to a process of manufacturing a cosmetic composition, as described above, said method comprising mixing the powder composition according to any one of the hereinabove described embodiments, with at least one cosmetically acceptable ingredient.

According to a last aspect, the invention relates to a process for preparing a powder composition according to the invention. The process comprises the steps of:
a. Supplying a hydrophobized inorganic powder composition comprising hydrophobized composite particles presenting an inorganic core and a coating of a hydrophobic composition, then
b. Contacting the hydrophobized inorganic powder composition of step a) with a hydrophilic composition comprising from 20% to 60% of hydroxypropylmethylcellulose in weight relative to the total weight of the hydrophilic composition as described in any one of the above embodiments; then
c. Optionally drying and/or grinding the composition obtained in step b), and
d. Recovering the powder composition according to the invention.

According to some embodiments, the hydrophobized inorganic powder composition according to step a) is commercially available such as for example the hydrophobized inorganic powders the VAA^{®}, NAI^{®}, VAI^{®} or MiyoNAT VAA^{®} product series commercialized by MIYOSHI.

According to some embodiments, the hydrophobized inorganic powder composition according to step a) is prepared *in situ* by:
a1. supplying an inorganic powder composition comprising inorganic particles as described above, then
a2. treating the inorganic particles of step a1) with a hydrophobic composition as described above
a3. optionally drying and/or grinding the composition obtained in step a2), and
a4. recovering a hydrophobized inorganic powder composition comprising hydrophobized composite particles presenting an inorganic core and a coating of a hydrophobic composition.

The hydrophobized inorganic powder obtained in step a4) can then be supplied according to the process step a) and be subjected to steps b)-d) of the powder manufacturing process according to the invention.

According to some embodiments, step b) leads to a weight increase of the hydrophobized inorganic particles of step a) ranging from 2% to 5%, typically about 3%.

According to some embodiments, step b) comprises mixing the hydrophilic composition in water to obtain an aqueous preparation of the hydrophilic coating composition, then suspending the hydrophobized inorganic powder in the aqueous preparation and stirring of the obtained suspension. In some embodiments, the hydrophilic coating composition is in an amount ranging from 15% to 30%, such as fro example about 20%, in weight relative to the total weight of the aqueous preparation of the hydrophilic coating composition.

Grinding according to steps a3) and c) can be carried out by any grinding means known in the art, such as milling.

Drying according to steps a3) and c) can be carried out by any drying means known in the art, such as air-drying or lyophilization.

### EXAMPLES

The present invention is further illustrated by the following examples.

### Example 1: Preparation of the hydrophilic coating composition

A hydrophilic coating composition according to the invention was prepared according to table 1.

**Table 1. Composition of the hydrophilic coating according to Example 1**

| | % in weight relative to the total weight of the coating composition |
|---|---|
| Hydroxypropylmethylcellulose | 50.0 |
| Magnesium carbonate | 30.0 |
| Microcrystalline cellulose | 10.0 |
| Hydroxypropylcellulose | 5.0 |
| Miglyol (medium chain triglycerides) | 5.0 |

### Example 2: Coating with the hydrophilic coating composition

The hydrophilic composition of example 1 was mixed with water to obtain an 20% aqueous preparation of the hydrophilic coating composition under continuous stirring.

Dry inorganic powders of titanium dioxide (306W), red iron oxide (306R), yellow iron oxide (306Y), black iron oxide (306B) were hydrophobized with 3 % of aluminum and/or sodium myristoyl glutamate.

The hydrophobized composite particles of these inorganic powders were then coated with the hydrophilic coating composition of example 1 under the following conditions.

Each of the hydrophobized inorganic powders was suspended in the aqueous preparation and stirred. Then, the obtained suspensions were dried in the oven followed by overnight air drying then and milled to obtain the following powder compositions according to the present invention.

**Table 2. Powder compositions according to the invention comprising composite particles of titanium and iron oxides, having a hydrophobic coating composition consisting of a myristoyl glutamate aluminum and/or sodium salt and a second hydrophilic coating according to table 1.**

| Hydrophobized particles | Inorganic core | % of hydrophilic coating relative to the weight of the final powder composition |
|---|---|---|
| 4% HPW-306W | Titanium dioxide (TiO₂) | 4% |
| 8% HPW- 306Y | yellow iron oxide (FeHO₂), | 8% |
| 8% HPW-306R | red iron oxide (Fe₂O₃), | 8% |
| 8% HPW-306B | black iron oxide (Fe₃O₄), | 8% |

Given that, the inorganic core of the particles of iron oxides and hydroxides present an average diameter ranging from 5 µm to 20 µm, while titanium dioxide (TiO₂) particles present an average diameter ranging from 100 nm to 1 µm, the amount of the hydrophilic coating composition was adapted as per in table 2.

### Example 3: Analysis of the obtained powder compositions

The powder compositions according to example 2 were subjected to a comparative sensorial analysis wherein three testers assessed the sensorial properties of the pigment compositions before and after the coating with the hydrophilic coating composition according to example 1. The results are presented in table 3.

**Table 3. Results of the comparative sensorial analysis between the powder hydrophobized compositions 306W-B and the powder compositions according to the invention further comprising a hydrophilic coating according to example 2 (table 2).**

| Hydrophobized and hydrophilized particles | Comparative Sensorial Analysis |
|---|---|
| 4% HPW-306W | Brighter white dry feeling - less greasy and sandy texture |
| 8% HPW-306Y | dry feeling - less greasy sandy feeling |
| 8% HPW-306R | dry feeling - less greasy and sandy feeling |
| 8% HPW-306B | Darker Black dry feeling - little greasy and sandy feeling |

All testers reported a dry feeling on the skin with the powder compositions according to the invention and a reduction of the greasy and sandy texture of the particles compared to the particles that were coated only with the hydrophobic coating composition.

The subsequent coating with the hydrophilic coating composition according to example 1 did not significantly impact the color tone of the pigment compositions. On the contrary the white titanium dioxide pigment composition according to the invention presented a brighter white tone compared to the creamy titanium dioxide pigment composition that was coated only with a hydrophobic composition. Likewise, the black iron oxide pigment composition according to the invention presented a darker black tone compared to the black iron oxide pigment composition that was coated only with a hydrophobic composition.

Furthermore, powder compositions 4% HPW-306W and 8% HPW- 306Y according to table 2 were subjected to a water affinity test.

Powder compositions 4% HPW-306W and 8% HPW- 306Y were suspended into water followed by adding an equal volume of isononyl isononanoate thereby forming a biphasic system.

After stirring and letting the biphasic system to set, it could be observed that both powders were retrieved in the lower water phase. After one month:
- no significant migration to the oil phase could be observed for either of the 4% HPW-306W and 8% HPW- 306Y powders
- both 4%HPW-306W and 8% HPW- 306Y powders presented some sedimentation in the water phase that was easily reconstituted by gentle shaking, and
- no particle aggregates were observed.

In view of the above, the powder composition according to the invention not only allows the improvement of the sensorial properties of skin-compatible hydrophobized pigment compositions but also paves the way to handling hydrophobized pigment particles in aqueous cosmetic compositions without the need to generate emulsions.

## Claims

1. A powder composition comprising composite particles, wherein the composite particles present an inorganic core, a hydrophobic coating composition and a hydrophilic coating composition, wherein the hydrophilic composition comprises from 20% to 60% of hydroxypropylmethylcellulose in weight relative to the total weight of the hydrophilic composition.

2. The powder composition according to claim **1,** wherein the coating of the hydrophilic composition coats the coating of the hydrophobic coating composition.

3. The powder composition according to claim **1** or claim **2,** comprising in weight relative to the total weight of the powder composition:
- from 0.5% to 15% preferably from 1% to 12%, of the hydrophobic coating composition,
- from 0.5% to 10%, preferably from 3% to 8%, of the hydrophilic coating composition.

4. The powder composition according to any one of claims **1** to **3,** wherein the hydrophilic coating composition further comprises microcrystalline cellulose, preferably in an amount ranging from 5% to 25% in weight relative to the total weight of the hydrophilic composition.

5. The powder composition according to any one of claims **1** to **4,** wherein the hydrophilic coating composition further comprises from 15% to 40% in weight relative to the hydrophilic composition of an inorganic filler selected from the group consisting of magnesium carbonate, calcium carbonate, titanium dioxide, silica, silica beads, mica, talc, sericite, and mixtures thereof, preferably the inorganic filler is magnesium carbonate.

6. The powder composition according to any one of claims **1** to **5,** wherein the hydrophilic composition comprises in weight relative to the total weight of the hydrophilic composition:
- from 30% to 55%, preferably from 45% to 55%, of hydroxypropylmethylcellulose,
- from 5% to 25%, preferably from 8% to 12%, of microcrystalline cellulose, and
- from 15% to 40%, preferably from 25% to 35%, of magnesium carbonate.

7. The powder composition according to any one of claims **1** to **6,** wherein the hydrophilic composition further comprises at least one triglyceride, hydroxypropylcellulose or mixtures thereof.

8. The powder composition according to any one of claims **1** to **7,** wherein the hydrophilic composition comprises in weight relative to the total weight of the hydrophilic composition:
- from 45% to 55%, of hydroxypropylmethylcellulose,
- from 8% to 12%, of microcrystalline cellulose,
- from 25% to 35%, of magnesium carbonate,
- from 1% to 10%, of hydroxypropylcellulose, and
- from 1% to 10%, of at least one triglyceride.

9. The powder composition according to any one of claims **1** to **8,** wherein the hydrophobic composition comprises:
- at least one N-acylamino amino acid or a salt thereof, wherein the acyl is a C12-C18 acyl group,
- at least a silicone surface agent, or
- mixtures thereof.

10. The powder composition according to any one of claims **1** to **9,** wherein the hydrophobic coating composition comprises at least one N-acylamino amino acid or a salt thereof, preferably selected from stearoyl glutamine, palmitoyl glutamine, myristoyl glutamine, sodium salts thereof, aluminum salts thereof or mixtures thereof.

11. The powder composition according to any one of claims **1** to **10,** wherein the inorganic core is selected from the group consisting of titanium dioxide, red iron oxide, yellow iron oxide, black iron oxide, mica, nacre, chromium oxide, ultramarine blue, ferric blue, manganese violet, zinc oxide, aluminum oxide, zirconium oxide, boron nitride, barium sulfate or mixtures thereof.

12. A cosmetic composition comprising the powder composition according to any one of claims **1** to **11.**

13. The cosmetic composition according to claim **12,** wherein the cosmetic composition is selected from a sunscreen composition, a facial make-up composition, a cosmetic foundation composition or a hair make-up composition.

14. A process for preparing a powder composition according to any one of claims **1** to **11,** said process comprising the steps of:
a. Supplying a hydrophobized inorganic powder composition comprising hydrophobized composite particles presenting an inorganic core and a coating of a hydrophobic composition, then
b. Contacting the hydrophobized inorganic powder composition of step a) with a hydrophilic composition, wherein the hydrophilic composition comprises from 20% to 60% of hydroxypropylmethylcellulose in weight relative to the total weight of the hydrophilic composition; then
c. Optionally drying and/or grinding the composition obtained in step b), and
d. Recovering the powder composition according to any one of claims **1** to **11.**

15. The process according to claim **14,** wherein step a) comprises:
a1. supplying an inorganic powder composition comprising inorganic particles, then
a2. treating the inorganic particles of step a1) with a hydrophobic composition
a3. optionally drying and/or grinding the composition obtained in step a2), and
a4. recovering a hydrophobized inorganic powder composition comprising hydrophobized composite particles presenting an inorganic core and a coating of a hydrophobic composition.
